# EUROPEAN PATENT SPECIFICATION

(11) **EP 0 494 848 B1**
(45) Date of publication and mention of the grant of the patent: **26.03.1997**
(21) Application number: 92850001.6
(22) Date of filing: 03.01.1992
(51) Int. Cl.: A61K 31/155, A61K 31/195, A61K 31/16, C07C 279/14

(54) **Method of removing endotoxins**
Verfahren zur Entfernung von Endotoxinen
Méthode pour séparer les endotoxines

(30) Priority: 09.01.1991 SE 9100059
(43) Date of publication of application: 15.07.1992
(60) Divisional application: 95118986.9
(73) Proprietor: Pharmacia AB, 112 87 Stockholm (SE)
(72) Inventor: Grimfors, Christer, S-182 75 Täby (SE); Lampén, Ellinor, S-163 65 Spanga (SE); Lindgren, Svante, S-195 00 Märsta (SE); Wahlén, Raymond, S-194 53 Upplands Väsby (SE); Sandberg, Göran, S-762 00 Rimbo (SE); Westberg, Björn, S-172 65 Spanga (SE)

(56) References cited:
- EP-A- 0 333 474
- EP-A- 0 342 139
- GB-A- 1 253 830
- GB-A- 2 240 041
- US-A- 4 282 217
- US-A- 4 308 280
- US-A- 4 405 643
- STN International, File Medline, STN accession no. 84040139; G.L. KOVACS et al.: "Hormonally active arginine-vasopressin suppresses endotoxin-induced fever in rats: lack of effect of oxytocin and a behaviorally active vasopressin fragment", Neuroendocrinology, vol 37, no 4, October 1983
- STN International, File Medline, STN accession no. 88290981; J.V. REYNOLDS et al.: "Immunomodulatory mechanisms of arginine", Surgery, vol 104, no 2, August 1988
- STN International, File BIOSIS, STN accession no. 91:55734; R.G. KILBOURN et al.: "Reverseal of endotoxin-mediated shock by N-G methyl-L-arginine an inhibitor of nitric oxide synthesis", Biochem. Biophys. Res. Commun., vol 172, no 3, 1990

## Description

When manufacturing pharmaceuticals for parenteral use, one of the most important prerequisites is that the products included in the pharmaceutical are non-pyrogenic, i.e. that the endotoxin concentration of the pharmaceutical concerned is so low that only very small biological effects or no biological effects can be detected with conventional test systems (limulus tests = LAL or temperature increase in rabbits). Endotoxins are high molecular complexes associated with the outer cell wall of Gram-negative bacteria (e.g. E. Coli, Proteus or Salmonella), from which lipopolysaccharides (LPS) can be released (endotoxins, O-antigens) (Rietschel, E.T., et al, in Bacterial Endotoxins: Structure, Biomedical Significance and Detection with Limulus Amebocyte Lysate Test, pages 31-50, Alan R. Liss Inc., 1985).

Endotoxins are present in and are often the cause of the clinical symptoms in sepsis and in ARDS and DIC (adult respiratory distress syndrome and direct intravascular coagulation respectively) (Zaren, B. and Hedstrand, U., Intensivvård, pages 63-64, Uppsala University, Reprocentralen HSC, 1989).

Subsequent to having treated patients suffering from, e.g., septicemia with antibiotics, it is well known that the temperature of the patient will rise or that a further fever peak will occur, so-called Herxheimer's reaction, wherewith dead bacteria and parts thereof, including endotoxins, enter the blood circulation.

Clinical signs of the effect of endotoxins (the limit at which these can be shown is about 5 EU per kilo of body weight in rabbits and human beings) can sometimes be observed when pharmaceuticals and nutrient solutions are administered parenterally. In the case of human beings and rabbits for instance, the clinical signs are manifested by a feverish state, due to the ability of the endotoxins to release endogenic pyrogens which influence the thermoregulatory centre in the central nervous system. Other manifestations can also be observed in the central nervous system (Nowotny, B., Naturwissenschaft 58, pages 397-409, 1971). Such cardiovascular changes as hypotension and permeability changes in arteriole and venules, for instance, may explain certain important organ changes which often occur in Gram-negative sepsis (Zaren, B. and Hedstrand, U., Intensivvård, pages 63-64; Uppsala University, Reprocentralen HSC, 1989; Nowotny, B., Naturwissenschaft 58, pages 397-409, 1971; Gilbert, R.P., Physiol. Rev. 40, 245, 1960; Vick, J.A., Am. J. Phys. 200, 944, 1964).

Those depyrogenizing methods which can be applied in vitro today are based on two principle techniques, namely a) to guard against endotoxin contamination and b) to remove endotoxins during formulation.

It is difficult to carry out the first method a) strictly, because it is necessary for aseptic conditions to prevail during the whole of the formulating process and also during the preparation of starting materials. The second method b) has resulted in the development of different filtering methods, these methods including the use of asbestos filters, ion exchangers, and have involved adsorption on activated carbon or on barium sulphate suspensions, gamma radiation, filtration through membranes having an exclusion limit ranging from 100,000 Daltons to 0.1 micron of endotoxin aggregate, the supply of amebocytlysate and the removal of the gel formed, and also the use of ultrafilters having an exclusion limit of 10,000 Daltons for filtering-out non-aggregated endotoxins. At the present time, ultrafiltration is primarily applied industrially, whereas the other methods have been abandoned, with the exception of asbestos filtration. Two depyrogenizing methods, namely autoclaving alone or in combination with extremely low pH-values now have limited value because of their low efficiency and because of damage caused to the products (Mosier, L.D., et al. J. Parent. Sci. and Technol., Vol. 41, No. 1, pages 21-25, 1987). The ultrafiltration method, however, results in high production costs, because of the expensive material and high working costs involved. Furthermore, the equipment used is often highly space-consuming and often of doubtful efficiency, resulting in floating exclusion limits and enabling endotoxins to pass through the filters to some extent.

One particular problem in this regard is the assaying of endotoxins in biologically active substances, such as coagulation factor 2 (prothrombin) for instance, or when the sample material is highly restricted but has a very high biological potency, there excluding the use of both the limulus test and experimental animals.

So-called plasmapheresis and hemoperfusion through filters that contain an immobilized product of polymyxin B have been tested in vivo for the purpose of removing endotoxins from the blood path.

European Patent Specification No. EP-A-0333474 relates to selective removal of endotoxins from endotoxin-contaminated aqueous solutions specifically containing physiologically-active high-molecular weight substances (valuable proteins) for injection or parenteral administration. The solution is contacted with an adsorbent capable of specifically adsorbing the protein thereon. The immobilized protein is contacted with a solution containing an amino compound, e.g. arginine, for selectively removing the endotoxins, while the protein is eluted from the adsorbent.

### Description of the Invention

The invention relates to a method of removing endotoxins from water, pharmaceutically useful solutions, pharmaceutical preparations, plasma or blood, said method comprising filtering the water, pharmaceutically useful solutions, pharmaceutical preparations, plasma or blood through a bed which contains an immobilized compound according to formula (I) 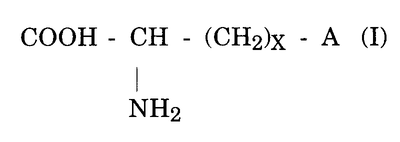 in which X is an integer of from 2 to 5 and
A signifies - NH - C(=NH) - NH₂ or - CH₂ - NH₂.

The pharmaceutical preparation may contain a biologically active component, such as a coagulation factor for instance.

The invention also includes the use of an immobilized compound according to formula (I) for removing endotoxins from water pharmaceutically useful solutions, pharmaceutical preparations, plasma or blood, and also a method of enriching endotoxins, said method comprising passing water or a pharmaceutical solution containing endotoxins through a column which contains an immobilized compound according to formula (I).

By "arginine or structurally related substances" is meant in the following compounds according to formula (I).

Other features of the invention will be apparent from the following description and from the claims.

The use of the inventive compounds will now be exemplified with the aid of a number of test examples.

Immobilized arginine in the form of Arginin-Sepharose® (Kabi Pharmacia Fine Chemicals) was used experimentally to bind endotoxins in aqueous solution with the intention of further evaluating the binding ability of the endotoxins with the aid of in vivo and in vitro techniques. Test Examples 1 and 2.

### Test Example 1

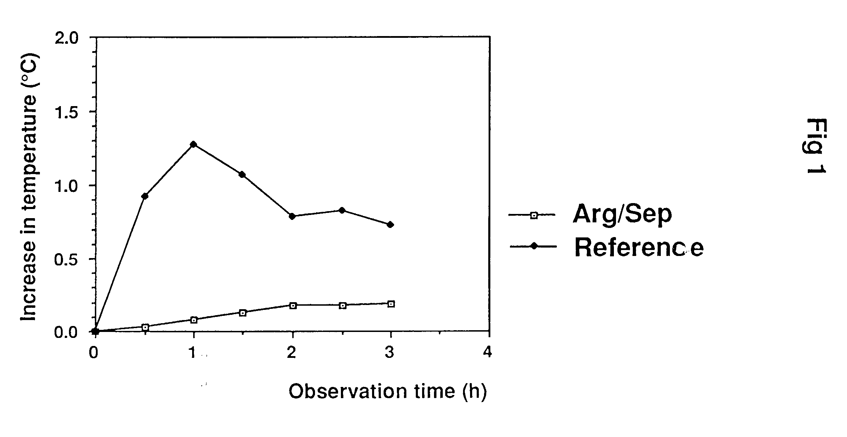

A small amount of chemically pure glass wool was inserted into each of six Pasteur pipettes to form a column packing. The resultant columns were washed with 6 M hydrochloric acid three times and then with sterilized water and absolute alcohol to obtain a neutral reaction. The columns were then dried at 180°C for four hours in a heated cabinet. 1 ml of Arginin-Sepharose® (gel for affinity chromatography from Kabi Pharmacia Fine Chemicals) to each of these columns. The columns were then washed with 10 total volumes of sterilized water, whereafter 2000 EU of endotoxins from E. Coli were introduced to the columns and allowed to drip therethrough. 1 ml of sterilized water was then introduced into each of said columns, this water also being allowed to drip through the columns. The water that had passed through respective columns was collected (a total of 1.5 ml was collected from the columns, i.e. an amount sufficient to cover the total volume plus the void volume). A physiological saline solution (0.9% sodium chloride solution) was then added to this liquid, so as to obtain a volume of 40 ml. Each of six rabbits was administered intravenously with 10 ml of this mixture for each kilo of body weight and the temperature of the rabbits was recorded once every thirty minutes with the aid of a rectally applied constant-recording analogue temperature probe. Each of six further rabbits were administered intravenously with 10 ml of a physiological sodium chloride solution for each kilo of body weight, said sodium chloride solution being admixed with 500 EU endotoxin per kilo of body weight. The endotoxin was taken from the same batch as that mentioned above. The temperature was measured in the same manner as that aforedescribed. These latter rabbits were used as reference animals (see Figure 1 and Tables 1 and 2). A limulus test for endotoxins was carried out on those liquids that had passed through the six Arginin-Sepharose® columns. The solution from all six beds or columns showed a negative result, i.e. the endotoxin concentration did not exceed the detection limit for this system (0.12 EU).

### Table 1

Temperature change in rabbits over a period of 3.0 hours subsequent to injecting endotoxin solution, corresponding to 500 EU/kg body weight which had passed through an Arginine-Sepharose® bed.

A solution of an equivalent amount of endotoxins with an 0.9% saline solution was used as a reference substance.

The mean value disclosed in the Table relates to the area between the line of the initial temperature and the fever chart for n number of observations.

| Group | Mean Value ± SEM | n |
|---|---|---|
| a. Arginine-Sepharose® | 0.34 ± 0.12 | 6 |
| b. Reference | 2.61 ± 0.24 | 6 |
| a/b p<0.05 | | |

### Table 2

Maximum rise in the rectal temperature of rabbits subseqeunt to injecting endotoxin solution corresponding to 500 EU/kg body weight that had passed through an Arginine-Sepharose® bed.

Physiological saline solution in which an equivalent amount of endotoxins (500 EU) had been dissolved was used as a reference substance.

The Table shows the mean value of n number of observations.

| Group | Mean Value ± SEM | n |
|---|---|---|
| a. Arginine-Sepharose® | 0.24 ± 0.05 | 6 |
| b. Reference | 1.33 ± 0.06 | 6 |
| a/b p<0.05 | | |

### Test Example 2

A separate experiment was carried out in vitro using five different columns which contained endotoxins bound to immobilized arginine in the form of Arginine-Sepharose® (Kabi Pharmacia Fine Chemicals). These columns were prepared in accordance with the aforedescribed Example 1. The columns were washed with sterilized water, whereafter 1400 EU of endotoxins obtained from E. Coli were dripped through the columns. The columns were then eluted with 2 ml of a physiological saline solution (0.9%), whereafter the concentration of endotoxins in the eluate was determined with the aid of a limulus test. This test was chosen because it is an accepted method (Ph. Eur., V. 2.1.9.) and because the method shows the presence of endotoxins in the solution clearly. The concentration of active endotoxins was measured in the eluate obtained from all five columns and was found to be >110 EU/ml. A further elution was carried out with 2 ml of a 1.8% sodium chloride solution and the endotoxin-concentration of the eluate from all five columns was determined and found to lie within the range of 110-220 EU/ml.

The experiment showed that endotoxins bonded to the Arginine-Sepharose® in the column and that these bonds could be broken by eluting with a saline solution (0.9 or 1.8%).

It is evident from the experiments disclosed in the Test Examples that:
- Arginine in an immobilized form has an affinity to and effectively binds endotoxins in vitro (see Tables 1 and 2).
- Endotoxins are bound to Arginin-Sepharose® and can be eluted therefrom.

The temperature inhibition corresponds to a general endotoxin inhibition, as the immobilizing experiment with Arginine-Sepharose® indicates very clearly.

Under the aforesaid conditions, endotoxins can also be removed by hemofiltration, using a filter which contains immobilized arginine or structurally-related substances. Such filters can also be used to remove pyrogens from distilled water in the manufacture of pharmaceutical preparations intended for intravenous, intramuscular, intracutaneous or intraperitoneal use, and can also be removed from the pharmaceutical preparations themselves. Immobilized arginine or structurally-related substances can also be used to enrich endotoxins for further quantative determination from such solutions as those which are biologically highly active, for example coagulation factor 2 (prothrombin), and factors 8, 9 and 10. The same method can also be used to remove endotoxins from solutions intended for parenteral use.

Uremia patients who undergo hemodialysis represent a large area in which immobilized arginine or structurally-related substances can be used. These patients relatively often suffer from endotoxin effects, due to the endotoxins penetrating the dialysis filters.

## Claims

1. A method of removing endotoxins from water, pharmaceutically useful solutions, pharmaceutical preparations, plasma or blood, **characterized** by filtering the water, pharmaceutically useful solutions, pharmaceutical preparations, plasma or blood through a bed which contains an immobilized compound according to formula (I) 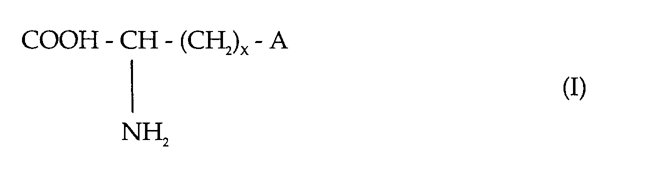 in which x is an integer of from 2 to 5 and
A signifies - NH - C(= NH) - NH₂ or - CH₂ - NH₂.

2. A method according to claim 1, **characterized** in that the pharmaceutical preparations contain a biologically active component.

3. A method according to claim 2, **characterized** in that the biologically active component is a coagulation factor.

4. The use of an immobilized compound according to formula (I) in which x is an integer of from 2 to 5 and A signifies -NH-C(=NH)-NH₂ or -CH₂-NH₂ for removing endotoxins from water, solutions for pharmaceutical use, pharmaceutical preparations, plasma or blood.

5. A method of enriching endotoxins, **characterized** by passing water or a pharmaceutical solution containing endotoxins through a column with an immobilized compound according to formula (I) in which x is an integer of from 2 to 5 and A signifies -NH-C(=NH)-NH₂ or -CH₂-NH₂.

## Patentansprüche

1. Verfahren zum Entfernen von Endotoxinen aus Wasser, pharmazeutisch geeigneten Lösungen, pharmazeutischen Zubereitungen, Plasma oder Blut,
gekennzeichnet durch Filtrieren des Wassers der pharmazeutisch geeigneten Lösungen, pharmazeutischen Zubereitungen, von Plasma oder Blut durch ein Bett, enhaltend eine immobilisierten Verbindung der Formel (I) 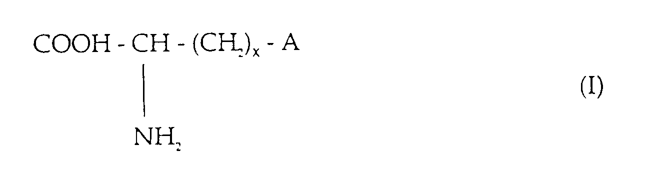 in der x eine ganze Zahl von 2 bis 5 ist und A -NH-C(= NH)-NH₂ oder -CH₂-NH₂ bedeutet.

2. Verfahren nach Anspruch 1,
dadurch gekennzeichnet, daß die pharmazeutischen Zubereitungen eine biologisch aktive Komponente enthalten.

3. Verfahren nach Anspruch 2,
dadurch gekennzeichnet, daß die biologisch aktive Komponente ein Koagulationsfaktor ist.

4. Verwendung einer immobilisierten Verbindung der Formel (I), in der x eine ganze Zahl von 2 bis 5 ist und A -NH-C(= NH)-NH₂ oder -CH₂-NH₂ bedeutet, zur Entfernung von Endotoxinen aus Wasser, Lösungen zur pharmazeutischen Verwendung, pharmazeutischen Zubereitungen, Plasma oder Blut.

5. Verfahren zur Anreichung von Endotoxinen, dadurch gekennzeichnet, daß man Wasser oder eine pharmazeutische Lösung, enthaltend Endotoxine, durch eine Säule leitet mit einer immobilisierten Verbindung der Formel (I), in der x eine ganze Zahl von 2 bis 5 ist und A -NH-C(= NH)-NH₂ oder -CH₂-NH₂ bedeutet.

## Revendications

1. Procédé destiné à séparer les endotoxines de l'eau, de solutions pharmaceutiquement utiles, de préparations pharmaceutiques, du plasma ou du sang, caractérisé par le fait de filtrer l'eau, les solutions pharmaceutiquement utiles, les préparations pharmaceutiques, le plasma ou le sang à travers un lit qui contient un composé immobilisé selon la formule (I) 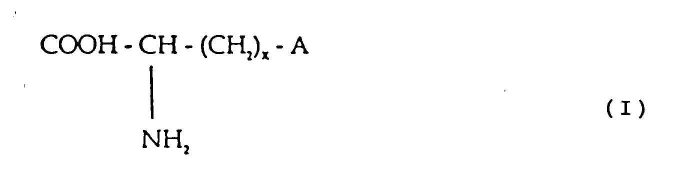 dans laquelle x est un nombre entier de 2 à 5 et A signifie - NH - C(=NH) - NH₂ ou - CH₂ - NH₂.

2. Procédé selon la revendication 1, caractérisé en ce que les préparations pharmaceutiques contiennent un composant biologiquement actif.

3. Procédé selon la revendication 2, caractérisé en ce que le composant biologiquement actif est un facteur de coagulation.

4. Utilisation d'un composé immobilisé selon la formule (I) dans laquelle x est un nombre entier de 2 à 5 et A signifie - NH - C(=NH) - NH₂ ou - CH₂ - NH₂ pour séparer les endotoxines de l'eau, des solutions pour usages pharmaceutiques, des préparations pharmaceutiques, du plasma ou du sang.

5. Procédé destiné à l'enrichissement d'endotoxines, caractérisé par le fait de faire passer de l'eau ou une solution pharmaceutique contenant des endotoxines dans une colonne avec un composé immobilisé selon la formule (I) dans laquelle x est un nombre entier de 2 à 5 et A signifie - NH - C(=NH) - NH₂ ou - CH₂ - NH₂.
